(19) Europäisches Patentamt
European Patent Office
Office européen des brevets

(11) **EP 4 464 339 A1**

(12) **EUROPEAN PATENT APPLICATION**
published in accordance with Art. 153(4) EPC

(43) Date of publication:
**20.11.2024 Bulletin 2024/47**

(21) Application number: **23740482.7**

(22) Date of filing: **12.01.2023**

(51) International Patent Classification (IPC):
***A61K 49/00*** (2006.01)   ***A61B 90/00*** (2016.01)

(52) Cooperative Patent Classification (CPC):
**A61B 90/00; A61K 49/00**

(86) International application number:
**PCT/KR2023/000601**

(87) International publication number:
**WO 2023/136639 (20.07.2023 Gazette 2023/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.01.2022 KR 20220005362**
**19.12.2022 KR 20220178024**

(71) Applicant: **National Cancer Center**
**Goyang-si, Gyeonggi-do 10408 (KR)**

(72) Inventors:
- **KIM, Seok Ki**
  **Seoul 02831 (KR)**
- **LEE, Sang Hyuk**
  **Daegu 41143 (KR)**

(74) Representative: **Hoefer & Partner Patentanwälte mbB**
**Pilgersheimer Straße 20**
**81543 München (DE)**

(54) **BIOCOMPATIBLE COMPOSITION FOR LABELING TARGET LESION, AND PREPARATION METHOD THEREFOR**

(57) The disclosure relates to a composition for labeling a target lesion, in form of microspheres including a biocompatible polymer; and a dye for biological tissue, and directly administered to a target lesion to confirm size and location of the target lesion in real time during surgery at a target site and a method preparing the same. The composition for labeling a target lesion of the present disclosure, when injected into a target lesion, remains there for several months and can detect the location and size of the labeled lesion in real time during surgery, thereby not only improving the success rate of surgical operation for the target lesion, but also preventing excessive loss of normal tissue, and therefore can be widely utilized as an effective surgical marker.

FIG. 1

## Description

### Technical Field

The present invention relates to a biocompatible composition for labeling a target lesion and a method for preparing the same, and in particular, to a composition for labeling a target lesion including a dye for biological tissue and a biological protein in a biocompatible polymer, a method of imaging information of labeling at a site of lesion using the composition for labeling a target lesion, and a surgical marker for a target lesion comprising a mixture of the composition for labeling a target lesion and a biopolymer in order to improve in vivo stability and imaging ability.

### Background Art

In the case of cancer treatment, methods using various anticancer drugs are being developed, but the most commonly used method is still surgical removal of cancer cells. However, when using surgical methods, techniques to minimize the extent of surgery during the operation are essential to ensure the patient's health and welfare after surgery. In particular, in the case of breast cancer, the goal of breast-conserving therapy can be achieved if fewer lesions are resected in Korean women with smaller breasts than in other countries. The extent of surgery is determined by the lesion and the extra marginal area therearound. If the surgeon does not know the exact location and extent of the lesion, he or she has no choice but to leave a large margin around the lesion. This is because if the surgeon reduces the surgical scope without precise knowledge, the tumor may remain in the resection margin. However, in actual clinical surgery, there are not many ways for the surgeon to accurately check the lesion in real time during surgery. Although highly precise diagnostic methods have been developed, they cannot be used during surgery, and during actual surgery, the surgeon mainly relies on their sense of touch or vision. In this case, it is rare for lesions to be clearly distinguished. In particular, small lesions are even more difficult to distinguish. In order to achieve the purpose of micro-invasive surgery and surgery, there is a need for a technology that informs the surgeon of the lesion during surgery.

In conventional tumor removal surgery, especially breast cancer surgery, the location of the patient's micro lesion is confirmed using ultrasound, mammography, or magnetic resonance imaging before surgery, the location of the confirmed lesion is labeled, and tissue is removed from the labeled area. Methods for labeling the location of a confirmed lesion include drawing a picture on the skin surface, using a wire, or injecting a black dye such as charcoal. However, the method of drawing with a pen on the skin to label the location of the lesion is the easiest to use, but has the disadvantage of being the least accurate, due to the highly flexible nature of breast tissue, the fact that the shape of the breast changes a lot during diagnosis and surgery, and in the case of lesions in the deep part of the breast, labeling only on the skin surface is insufficient. Next, the method of piercing a breast lesion using a wire has the disadvantages: it originally requires the wire to be inserted vertically into the skin surface, but since it may affect the ultrasound probe, it must inevitably be inserted diagonally; the position of the wire may move according to the movement of the breast, resulting in lower accuracy than expected; the inserted wire interferes with the surgery; and additional excision of the insertion site is required. Lastly, the method of injecting a dye such as charcoal has the advantage that the injected dye binds to the lesion and can accurately label the location of the lesion, but it has the disadvantage that, in the case of deep breast lesions, the black dye cannot be confirmed from the outside, and the surgical site may be contaminated by dye. The disadvantages listed above may also be commonly applied to surgery for the removal of cancerous tissue as well as breast cancer.

As such, it is difficult to precisely determine the surgical range for surgically removing cancerous lesions with the technology developed to date, so when cancerous lesions are removed surgically, there is no choice but to extend the resection range more than necessary. Even after surgery, tests must be conducted to confirm whether the lesion has been removed completely.

Under this background, the present inventors have confirmed that a marker (KR-10-2012-0153793), which uses a complex of macroaggregated albumin and a dye for biological tissue as a marker when surgically removing cancerous lesions, is effectively adsorbed to cancerous lesions to accurately indicate the location of the lesion, and that the scope of the lesion to be removed can be accurately identified by tracking the dye in real time during surgery.

In the case of general dyes, dyes or fluorescent dyes do not stay at the injection site for a long time, but spread to the surrounding area within a few hours, and are eventually excreted into the body, thus losing their ability to label lesions. In the case of the existing invention (KR-10-2012-0153793), it stays at the injection site longer than existing general (fluorescent) dyes, but it is difficult to exceed several days. This is especially true when the injection site is a tissue that is constantly moving or has high blood flow, such as the stomach, or where the tissue is relatively loose and not hard. This means that the conventional marker had an issue in that the dye did not stay at the injection site for a long time and spread to the surrounding area within a few hours, so the ability to label the lesion was lost in a short period of time.

For cancer patients with large lesions that cannot be operated on immediately, the use of neoadjuvant chemotherapy, in which chemotherapy is first administered followed by surgery to remove the cancer, is becoming more common. At this time, lesions are labeled in advance before chemotherapy, which takes several months, and these areas are excised after chemotherapy. If the tumor is successfully treated with chemotherapy and shrinks to a microscopic size, it is often difficult to find the cancer site during surgery. The cancer site is identified by placing a metal clip during chemotherapy, but it is not easy to find the metal clip during actual surgery, and it is not uncommon for it to move during a long chemotherapy period, so improvement measures are needed. It is very useful if the fluorescent pigment can remain in the injected area for several months and the fluorescence or color can be confirmed several months later.

In order for the (fluorescent) dye to stay at the injection site for a long time, it must be particles of an appropriate size or bound to such particles so that they do not spread to the surroundings (diameter: several to hundreds of micrometers), and the dye must be decomposed or denatured by biological reactions to be stable for a long period of time to produce color. However, it must not lose its fluorescence.

Microspheres of typical micrometer-sized particles have been developed from a variety of materials. A variety of porous microspheres have been developed as microspheres capable of carrying (fluorescent) dyes, but these are mainly used to release the loaded material, here (fluorescent) dyes, slowly and at a controlled rate. The main purpose is not to not excrete the drug, but to excrete it appropriately, which is completely different from the required purpose. In other words, the (fluorescent) dye must be in a form that does not escape from the microspheres into the biological body, and the microspheres must be made of materials that can be used in biological organisms.

In light of these considerations, the easiest method to devise is to mix biocompatible polymers with (fluorescent) dyes to make microspheres, but generally, heat and radicals are generated during the polymerization process synthesized from monomers, and the (fluorescent) dye is denatured, causing them to lose their color and fluorescence, thus defeating the purpose.

The present invention has devised a method of dissolving a biocompatible polymer in a solvent and completely embedding and encapsulating a (fluorescent) dye using a double emulsion method, and the dye encapsulated in appropriately prepared biocompatible polymer microspheres does not flow out, thereby solving the aforementioned disadvantages.

In addition, it also devised a method to allow the encapsulated (fluorescent) dye to be stabilized within the polymer microspheres for a long period of time, and it was confirmed that color or fluorescence could be maintained for a longer period of time if the dye is combined with proteins rather than the dye alone.

In addition, if the surface is modified with hyaluronic acid, it can activate fibroblasts in the surrounding tissue after injection to produce collagen tissue for better adhesion. However, hyaluronic acid is insoluble in organic solvents, making it difficult to coat polymeric microparticles with hyaluronic acid by conventional methods. By devising a method of dissolving hyaluronic acid or collagen in a water-soluble solvent during the process of preparing microparticles, the present invention has developed a preparation method that allows coating with hyaluronic acid or collagen in the process of making biocompatible polymeric microparticles.

(Prior art literature)

(Patent document)

Patent Document 1: KR 10-2012-0153793

## Disclosure

## Technical Problem

To solve the problems described above, an aspect of the present invention is to provide a composition for labeling a lesion in the body containing a biocompatible polymer synthetic material including a dye, fluorescent dye, or bioprotein.

Specifically, the purpose of the present invention is to (a) provide a method of labeling lesions in the body with a color or fluorescent dye that remains at the site of injection for several months or more; (b) provide a method of preparing a composite for labeling a lesion in the body and a composition of the injection; and (c) provide a method of using the composite to provide information about the location of the lesion.

## Solution to Problem

In order to achieve this purpose, in one general aspect, the present invention provides a composition for labeling a target lesion, in form of microspheres including a biocompatible polymer; and a dye for biological tissue, and directly administered to a target lesion to confirm size and location of the target lesion in real time during surgery at a target site.

The microsphere may further include a surface modifier coated on surface of the microsphere.

The biocompatible polymer may be one of polymethylmethacrylate (PMMA), polyglycolic acid (PGA), polylactic acid (PLA), polylactic acid-polyglycolic acid copolymer (PLGA), poly-$\varepsilon$-caprolactone (PCL), lactic acid-$\varepsilon$-caprolactone

copolymer (PLCL), polydioxanone (PDO), polytrimethylene carbonate (PTMC), polyamino acid, polyanhydride, polyorthoester, polyphosphazene, polyiminocarbonate, polyphosphoester, polyhydroxyvalate, copolymers thereof, and mixtures thereof.

The dye for biological tissue may be a visible dye or a fluorescent dye.

The visible dye may be one selected from a group of neutral red, Nile blue, Bismarck brown, lithium carmine, trypan blue, Janus green, methyl violet, auramine, malachite green, safranin, eosin, Congo red, erythrosine, nigrosine, Alcian blue hematoxylin, aniline blue, light green, and combinations thereof.

The fluorescent dye may be a near-infrared fluorescent dye.

The fluorescent dye may be an indocyanine green (ICG).

The fluorescent dye may be used alone or in conjugation with biological protein.

The surface modifier may be one of hyaluronic acid, collagen, and a mixture thereof.

The microsphere may further include biological protein.

The biological protein may be albumin.

The microsphere may have a diameter of 10 to 100 $\mu$m.

The composition may have an average density of 1 to 3 g/m$\ell$.

In order to achieve the above-mentioned purpose, in another general aspect, the present invention provides a method of preparing a composition for labeling a target lesion, including: mixing an organic solvent containing a biocompatible polymer and distilled water containing a dye for a biological tissue; and adding the mixed solution to a water-soluble solvent and stirring to form microspheres.

The method may further include adding a surface modifier to the water-soluble solvent.

The method may further include washing and filtering the stirred solution to select microspheres of a specific size.

The distilled water may further contain a complex of dye for biological tissue-biological protein.

The organic solvent may be one selected from a group of dichloromethane (DCM), o-xylene, m-xylene, p-xylene, and combinations thereof.

A concentration of the dye for biological tissue may be 1 to 100 $\mu$M.

The water-soluble solvent may be one selected from a group of polyvinylpyrrolidonem (PVP), polyacrylic acid (PAA), polyacryl amide (PAAm), polyhydroxyethyl methacrylate (PHEMA), polyvinyl alcohol, (PVA), and combinations thereof.

In order to achieve the above-mentioned purpose, in another general aspect, the present invention provides an injectable composition for labeling a target lesion, including a composition for labeling a target lesion in form of microspheres comprising a biocompatible polymer and a dye for biological tissues; and hyaluronic acid (HA) having an average molecular weight of 0.5 to 3.0 MDa.

With respect to 100 wt% of the injectable composition, hyaluronic acid may be included in an amount of 0.3 to 1 wt%.

The hyaluronic acid (HA) may have a viscosity of 24 to 1500 cps at room temperature.

A target lesion injected with the injectable composition may remain labeled for 4 to 12 months.

In order to achieve the above-mentioned purpose, in another general aspect, the present invention provides a method of providing information on location of a target lesion, including: directly administering a composition for labeling a target lesion in form of microspheres comprising a biocompatible polymer and a dye for biological tissue to a target lesion or around the target lesion; and recognizing a location of color change in a tissue to which the composition for labeling a target lesion as the target lesion to confirm a size and a location of the target lesion in real time during surgery.

**Effects of Invention**

The composition for labeling a target lesion of the present invention, once injected into the target lesion, can persist for up to several months, and the location, size, etc. of the labeled lesion can be detected in real time during surgery, which not only improves the success rate of surgical operation of the target lesion, but also prevents excessive loss of normal tissue, so it can be widely used as an effective surgical marker.

**Brief Description of Drawings**

FIG. 1 is a schematic diagram showing a method for preparing a biocompatible composition for labeling a target lesion of the present invention.

FIG. 2 is a schematic diagram detailing the preparation of the biocompatible composition for labeling a targeted lesion of the present invention.

FIG. 3 shows actual photographs of PMMA including ICG and HAS, photographs taken with a fluorescence camera, and photographs taken with a fluorescence microscope of PMMA with or without ICG-HAS binding.

FIG. 4 is a graph showing the density according to the concentration of the microspheres of the present invention in distilled water.

FIG. 5 is a graph showing the change in the near-infrared fluorescence signal intensity of microspheres according to the change in the concentration of ICG and albumin.

FIG. 6 is a photograph showing the degree of dispersion of microsphere particles according to the concentration of hyaluronic acid in the biocompatible injection composition for labeling target lesion of the present invention.

FIG. 7 is a graph showing a comparison of the change in intensity of near-infrared fluorescence signals over time for ICG, ICG-HSA, and ICG-HSA-PMMA particles under in vitro conditions, measured using IVIS (Perkin Elmer, Hopkinton, MA).

FIG. 8 is a photograph comparing the stability of the particles over time after placing the ICG-HSA-PMMA particles in HSA, distilled water, and agarose gel, respectively.

FIG. 9 is a comparison of the change in intensity of the near-infrared fluorescence signal over time when ICG-HSA-PMMA particles injected into ICR mice under in vivo conditions were injected together with hyaluronic acid or distilled water, as measured by IVIS.

FIG. 10 is a graph showing the change in intensity of the near-infrared fluorescence signal over time when ICG-HSA-PMMA particles were injected into ICR mice in vivo, together with hyaluronic acid or distilled water, and analyzed using the Image J program.

**Best mode for Implementation of the Invention**

Hereinafter, preferred embodiments of the present invention will be described in detail with reference to the accompanying drawings. Advantages and features of the present invention and a method of achieving them will become apparent with reference to the embodiments described below in detail with the accompanying drawings. However, the present invention is not limited to the embodiments disclosed below, but will be implemented in various forms, and only the present embodiments are provided to ensure that the disclosure of the present invention is complete, to fully inform those of ordinary skill in the art to which the present invention belongs, and the present invention is only defined by the scope of the claims. Hereinafter, the same reference numerals refer to the same elements.

In the present application, the term "comprise", "made of", or "include" is intended to specify that there is a feature, number, step, operation, component, part, or combination thereof described in the specification, and it should be understood not to preclude the presence or additional possibility of one or more other features or numbers, steps, operations, components, parts, or combinations thereof.

As used herein, the terms "about", "substantially", etc. are used to mean at or close to the numerical value when manufacturing and material tolerances inherent in the stated meaning are presented, and to aid understanding of the present application. It is used to prevent unscrupulous infringers from unfairly exploiting disclosures in which precise or absolute figures are mentioned. Additionally, throughout the specification herein, "step of" or "phase of" does not mean "step for."

Since those skilled in the art of the present invention can make various applications through the gist of the present invention, the scope of the present invention is not limited to the following examples. The scope of rights of the present invention extends to parts for which it is obvious that a person skilled in the art of the present invention can easily substitute or change the contents using prior art based on the matters stated in the patent claims.

Hereinafter, the present invention will be described in more detail with reference to the accompanying drawings where necessary.

**<Biocompatible composition for labeling target lesions>**

As a means to achieve the above-described object, the present invention provides a composition for labeling a target lesion which is in the form of microsphere particles comprising a biocompatible polymer; and a dye for biological tissue and is administered directly to the target lesion to confirm the size and location of the target lesion in real time during surgery at the target site.

The tissue labeled by the composition may preferably be solid tissue, such as solid cancer, into which the composition can penetrate and fix, and more preferably, may be prostate cancer, breast cancer, uterine cancer, skin cancer, cervical cancer, lung cancer, brain tumor, gastrointestinal tumor, liver cancer, soft tissue sarcoma, lymphoma, etc., but is not particularly limited to cancer or other benign lesions containing tissue into which the synthetic material can penetrate and fix.

The term "biocompatible polymer" of the present invention refers to a polymer that has no or little toxicity in the body and can be used for medical purposes, and an example thereof is "Poly(methyl methacrylate) (PMMA)". The biocompatible polymer of the present invention can be used to identify the size and location of lesion tissue by injecting it into the body to label it, and then remaining in the lesion for a long time.

The biocompatible polymer may be any one of polymethylmethacrylate (PMMA), polyglycolic acid (PGA), polylactic acid (PLA), polylactic acid-polyglycolic acid copolymer (PLGA), poly-ε-caprolactone (PCL), lactic acid-ε-caprolactone copolymer (PLCL), polydioxanone (PDO), polytrimethylene carbonate (PTMC), polyamino acid, polyanhydride, polyorthoester, polyphosphazene, polyiminocarbonate, polyphosphoester, polyhydroxyvalate, copolymers thereof, and mixtures thereof. Furthermore, the biocompatible polymer may be characterized in the present invention by embedding a dye or fluorescent dye and thus be transparent and thus have a light transmitting character.

The term "dye for biological tissue" in the present invention refers to a substance that binds to biological tissue and labels the location of the binding, so that the labeled location can be identified by the naked eye or by using a detection tool. The dye for biological tissue may be a visible dye or a fluorescent dye. For the purposes of the present invention, the dye for biological tissue may be included in a biocompatible polymer in a form that is bound to a bioprotein and used to label the lesion site, preferably, but not exclusively, a visible dye, a fluorescent dye that generates fluorescence at the binding site and can be detected using equipment, such as a fluorescence camera, either alone or in combination.

The term "visible dye" of the present invention refers to a type of dye that allows the labeled area to be identified with the naked eye by displaying a color in the visible light range when a labeling substance is bound to a biological tissue. For the purposes of the present invention, the visible dye can be injected into a lesion area in the body to clearly identify the lesion with the naked eye in the operating room, thereby improving the success rate of the surgery. The visible dye may be preferably neutral red, Nile blue, Bismarck brown, lithium carmine, trypan blue, Janus green, methyl violet, auramine, malachite green, safranin, eosin, Congo red, erythrosine, nigrosine, Alcian blue hematoxylin, aniline blue, light green, etc. alone or in combination, but is not particularly limited thereto, as long as they allow the target lesion tissue to be identified.

The term "fluorescent dye" of the present invention refers to an organic compound that absorbs light of a certain wavelength to form an excited state, and then generates fluorescence that maximizes the light penetration distance and minimizes error signals due to moisture. Preferably, it can be a near-infrared fluorescent dye, which is an organic compound that generates fluorescence of a near-infrared wavelength of 700 nm to 3000 nm, more preferably 750 nm to 900 nm. The fluorescence of the near-infrared wavelength generated from the above near-infrared fluorescent dye can be photographed or monitored in real time using equipment, such as a fluorescence camera, a fluorescence sensing probe (PCT/KR2011/009271), and a surgical fluorescence camera. The fluorescence of the near-infrared wavelength of the present invention has relatively low absorption in the body compared to other wavelengths, so that even near-infrared generated in a relatively deep part of the body can be detected outside the body. For the purpose of the present invention, the near-infrared fluorescent dye can be injected into a lesion site in the body and accurately identified in the operating room using fluorescent imaging equipment, thereby improving the success rate of the surgery. In particular, unlike the dye that can be identified with the naked eye, the location of the lesion can be detected even outside the body before incision and direct identification of the lesion, thereby facilitating rapid and accurate surgery. Preferably, indocyanine green (ICG) or the like can be used as the near-infrared fluorescent dye, and as long as it is a near-infrared fluorescent dye that can be used in the human body, it can be included in the scope of the present invention.

The complex in which the above near-infrared fluorescent dye is bound to a biocompatible polymer labels a target lesion, and exhibits the advantage of superior stability and accuracy of the detected fluorescent signal compared to the complex in which the near-infrared fluorescent dye is bound to other substances, so that the rate of detection of micro-lesions is high and the accuracy of lesion resection can be improved.

The term "indocyanine green (ICG)" of the present invention refers to a dye for fluorescent imaging in the near-infrared region that is widely used in the fields of biology or medicine. It has the characteristic of being decomposed and disappearing or being excreted through urine and feces after about an hour after being injected into the human body, so it is advantageous for clinical application as a fluorescent dye that can be used in the human body. In fact, cases of applying indocyanine green to the human body have been reported in several papers, and for example, it was reported that it was safely used clinically in 18 breast cancer patients (T. Kitai, et al., Breast Cancer, 12:211-215, 2005). It is already widely used clinically.

Fluorescent dyes, such as indocyanine green, are taken up by the liver from the tissues, decomposed, and excreted through the biliary tract. In order for dyes, such as indocyanine green, not to move to the veins or lymphatic vessels of the surrounding tissues, it is advantageous to bind to relatively large particles of a certain size (10 to 100 $\mu$m). The microspheres of the present composition can be manufactured in a form in which the fluorescent dye is completely encapsulated in the microspheres so that they can remain at the injection site for several months and continue to fluoresce. The water-soluble fluorescent dyes incorporated into the microspheres in this way are trapped in the walls of the hydrophobic polymer microspheres, so that the effect of remaining at the injection site for a long time can be expected.

In addition, even if the fluorescent dye is well encapsulated in the microsphere and does not flow to the surroundings, the dye should not be quenched by continuous excitation light or decomposed by a biological reaction and lose its properties as a dye. Here, quenching means that when the fluorescent dye is exposed to excitation light, it fluoresces in an excited state and falls to a stable state, but then decomposes due to the oxygen produced at that time or forms a diner (tetramer) and does not fluoresce, thereby losing its ability to generate fluorescence. If quenching occurs, even if the dye is at the injection site, it will not fluoresce and cannot serve as a marker. Therefore, in order for the fluorescent dye to be maintained for several months, it should be well bound, such as by being embedded in microspheres of an appropriate size so that the fluorescent dye, such as indocyanine green, stays at the injection site, and also should have little quenching.

Herein, the fluorescent dye can be used alone, or can be used by being conjugated with a biological protein. That is, in the composition for labeling a biocompatible target lesion of the present invention, the microsphere particles may further include a biological protein. When the fluorescent dye forms a conjugation with the biological protein, it can exhibit stronger fluorescence and a longer duration compared to when the above fluorescent dye is used alone. Here, the above biological protein may be, for example, albumin, but is not limited thereto, and can be included within the scope of the present invention as long as it forms a conjugation bond with the above fluorescent dye.

In addition, the composition for labeling the target lesion of the present invention may have a stronger fluorescence intensity as the bioprotein is included in a higher concentration. More specifically, the bioprotein may be included in the composition for labeling the target lesion at a concentration of 0.5 to 20 % w/v. Specific details regarding the bioprotein will be described in more detail below.

Furthermore, in the compositions for labelling lesions of the present invention, said microsphere particles may further comprise a surface modifier coated on the surface.

The above surface modifier may be any one of hyaluronic acid, collagen, and a mixture thereof. Since the microsphere particles containing the biocompatible polymer and the dye for biological tissue of the present invention remain in the body for a long time for the purpose of observing the lesion, side effects such as foreign body reactions by macrophages or foreign body giant cells may occur. Therefore, in order to reduce the occurrence of such side effects, hyaluronic acid or collagen, which are biopolymers widely present in the body, may be used as the surface modifier.

Here, the above microsphere particles may have a diameter of 10 to 100 $\mu$m. A more preferable diameter may be 30 to 70 $\mu$m, and an average diameter of 40 $\mu$m may be shown. The tissues of our body are densely composed of collagen fibers, reticular fibers, and elastic fibers with a diameter of tens to hundreds of nm, and the pore size of the capillary walls and lymphatic vessels passing into the tissue is also tens of nm. In order for the microsphere particles (ICG-albumin-PMMA microsphere) of the present invention not to flow out of the tissue, a micrometer size larger than the pore size of the fibers or capillaries/lymphatic vessels in the tissue is appropriate, and it was confirmed through animal experiments that the above particle size is effective. In order to obtain the above microsphere particle diameter, the microsphere particles may be obtained through filtering.

Here, the composition may have an average density of 1 to 3 g/m$\ell$. The density of this composition is higher than the density of distilled water at room temperature (0.99821 g/m$\ell$), and when diluted in distilled water for use as an injection, a problem of precipitation may occur. In the injection, the occurrence of such precipitation may cause a problem of clogging of the injection needle during injection or make it difficult to uniformly introduce the composition of the present invention to the target lesion. Therefore, the injectable composition including the composition for labeling the target lesion of the present invention may additionally be used by mixing the composition for labeling the target lesion with a biocompatible polymer such as hyaluronic acid in order to suppress the occurrence of precipitation. The specific details regarding the average density and the preparative composition of the composition for labeling the target lesion of the present invention will be described in more detail in the <Injectable Composition for Labeling the Target Lesion> and {Embodiment and Evaluation} paragraphs below.

**<Method for preparing a biocompatible composition for labeling a target lesion>**

The present invention provides a method for preparing a composition for labeling a target lesion as a means for achieving the above-described purpose. More specifically, the method for preparing a composition for labeling a target lesion of the present invention includes a step of mixing an organic solvent to which a biocompatible polymer is added and distilled water to which a pigment for biological tissue is added; and a step of mixing the mixed solution in a water-soluble solvent and then stirring to form microsphere particles.

In addition, the preparation method of the present invention may further include a step of adding a surface modifier to the water-soluble solvent. By further including the step as above, the surface modifier may be coated on the surface of the formed microsphere particles.

Here, the organic solvent may be any one selected from the group consisting of dichloromethane (DCM), o-xylene, m-xylene, p-xylene, and combinations thereof, but is not limited thereto.

Here, the water-soluble solvent may be any one selected from the group consisting of polyvinylpyrrolidone (PVP), polyacrylic acid (PAA), polyacryl amide (PAAm), polyhydroxyethyl methacrylate (PHEMA), polyvinyl alcohol (PVA), and combinations thereof, but is not limited thereto.

Here, the distilled water may be further supplemented with a dye for biological tissue-bioprotein conjugate.

In the method for preparing a biocompatible composition for labeling a target lesion of the present invention, the biocompatible polymer, the dye for biological tissue, the surface modifier, and the biological protein are the same as described above.

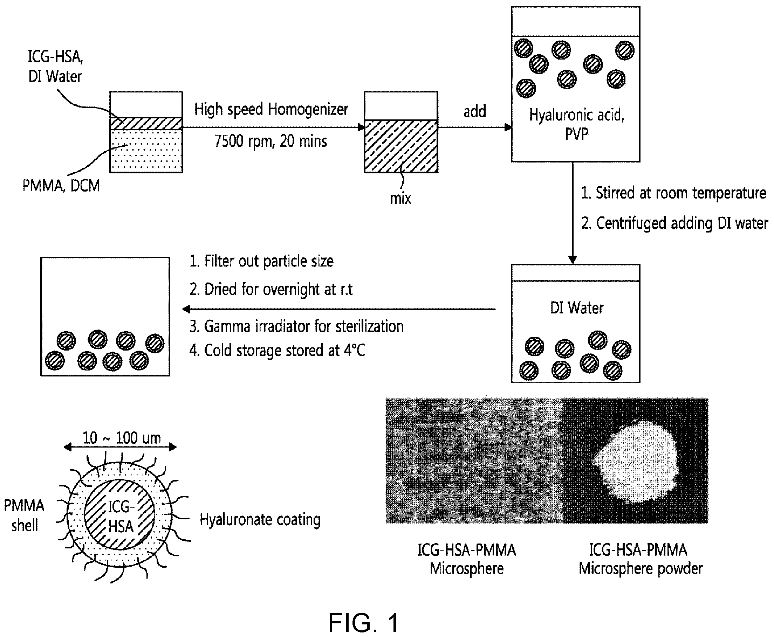

FIG. 1 is a schematic diagram showing a method for preparing a biocompatible composition for labeling target lesions of the present invention. More specifically, it shows a process for preparing a biocompatible composition for labeling target lesions using polymethyl methacrylate (PMMA) as the biocompatible polymer, indocyanine green (ICG) as the dye for biological tissue, hyaluronic acid as the surface modifier, and human serum albumin (HSA) as the biological protein.

Referring to FIG. 1, it can be confirmed that the biocompatible composition for labeling target lesions prepared according to the above-described preparation method appears in a spherical shape with a diameter size of 10 to 100 $\mu$m, and can be confirmed to appear in the form of a white powder when seen with the naked eye.

Here, the concentration of the dye for biological tissue may be 1 to 100 $\mu$M. A more preferable concentration may be 3 to 30 $\mu$M. In the present invention, as the concentration of the dye for biological tissue is included in the above-described range, the intensity of fluorescence may be stronger. Specific details related to the concentration of the dye for biological tissue will be described in more detail in {Embodiments and Evaluation} below.

Here, the concentration of the water-soluble solvent may be 0.1 to 10 % w/w, and the concentration of the surface modifier may be 0.01 to 0.3 % w/v.

In the present invention, in order to coat the above microsphere particles with hyaluronic acid or collagen, there may be a method of adding hyaluronic acid or collagen to the raw material of the polymer microspheres, but hyaluronic acid is not melted by heating, and there is a high possibility that hyaluronic acid will be denatured during the heating melting process. In addition, a method of dissolving the above hyaluronic acid in a solvent to create a hyaluronic acid solution and coating the microspheres with it may be considered, but the above hyaluronic acid does not dissolve well in general organic solvents, so it is impossible. In the present invention, a step of preparing a solution in which hyaluronic acid is well dissolved in the step of creating the microsphere particles was developed to provide a coating of hyaluronic acid. The above hyaluronic acid is known to have the effect of stimulating fibroblasts to produce collagen. The collagen produced in this way can be expected to have the effect of the composition for labeling target lesions injected into the tissue being well fixed to the injection site and not flowing away even after several months.

Here, the method for preparing the composition for labeling the target lesion may further include a step of washing and filtering the stirred solution to select microsphere particles of a specific size. As described above, in order for dyes such as indocyanine green not to move to veins or lymphatic vessels of surrounding tissues, it is preferable to bind to relatively large particles of a certain size (10 to 100 $\mu$m), so the method for preparing the composition for labeling the target lesion of the present invention may further include a step of washing and filtering the stirred solution to select microparticles of a size of 10 to 100 $\mu$m.

## <Injectable composition for labeling target lesion>

Furthermore, as a means for achieving the above-described object, the present invention provides an injectable composition for labeling a target lesion comprising a composition for labeling a target lesion in the form of microparticles containing a biocompatible polymer and a dye for biological tissue; and a hyaluronic acid (HA) having an average molecular weight of 0.5 to 3.0 MDa.

In order to maximize the usability of the biocompatible polymer containing the dye for biological tissue of the present invention as an in vivo marker, the physical properties of the composition for labeling the target lesion of the present invention can be enhanced by using the biopolymer. That is, the composition for labeling the target lesion of the present invention is injected into a lesion site in the body to label the lesion, thereby enabling clear recognition of the lesion site during surgery. In order for such a composition to smoothly perform the purpose as a marker described above, diffusion should be prevented as much as possible from the injected lesion site and a high fluorescence signal should be continuously emitted. "Hyaluronic acid" can be used as one means for achieving this purpose. When the composition for labeling the target lesion of the present invention is mixed with a hyaluronic acid solution, the in vivo residuality of the composition can be improved by the hyaluronic acid solution.

In addition, in the injectable composition for labeling the target lesion of the present invention, when the hyaluronic acid is additionally mixed into the injection, it can prevent the precipitation of microspheres by slowing down or blocking the precipitated microspheres, thereby preventing the injection failure due to the syringe needle being blocked by the precipitated microspheres. Specific details related thereto will be described in more detail in {Embodiments and Evaluation} below.

Here, hyaluronic acid (HA) may be included in an amount of 0.3 to 1 wt% based on 100 wt% of the injectable composition. If the hyaluronic acid is included in an amount of less than 0.3 wt%, the composition for labeling the target lesion may precipitate, causing a problem in which the syringe needle is clogged due to the precipitated composition. On the other hand, if the hyaluronic acid exceeds 1 wt%, the injectable composition may be excessively viscous, causing a problem in which inconvenience occurs during injection. Accordingly, it is preferable that the hyaluronic acid is included in an amount of 0.3 to 1 wt% based on 100 wt% of the injectable composition of the present invention. Specific details related thereto will be described in more detail in {Embodiments and Evaluation} below.

Here, the hyaluronic acid (HA) may have a viscosity of 24 to 1500 cps at room temperature. If the viscosity of the hyaluronic acid is less than 24 cps, the viscosity of the injectable composition of the present invention may be too low, making it difficult to control the injection amount during injection, or problems such as precipitation may occur. On the other hand, if the viscosity exceeds 1500 cps, excessive force applied to the syringe may be required due to the excessively high viscosity, which may cause problems such as difficulty in injection. Accordingly, it is preferable that the viscosity of the hyaluronic acid is 24 to 1500 cps at room temperature.

Here, the target lesion into which the above-mentioned injectable composition is injected may be labeled for several months or longer. More specifically, it may be desirable for the labeling of the target lesion through the above-mentioned injectable composition to be maintained for 4 to 12 months. After the above-mentioned composition is injected into the target lesion prior to pretreatment cancer treatment (pre-operative chemotherapy), the composition should continue to fluoresce even after the neoadjuvant chemotherapy so that the target lesion can be followed up. The specific duration of the above-mentioned neoadjuvant chemotherapy may vary depending on the type of cancer and the type of cancer treatment, and may change with the development of chemotherapy, but may generally be performed for a period of about 4 to 8 months. Accordingly, the target lesion into which the above-mentioned injectable composition for labeling the target lesion is injected is desirably labeled (fluorescent) for 4 months or longer, more desirably for 6 months or longer, and even more desirably for 8 months or longer. Specific details related to the stability of the above-mentioned injectable composition within the target lesion are described in more detail in {Embodiments and Evaluation} below.

**<Method of providing information on the location of the target lesion>**

In addition, the present invention provides a method for providing information on the location of a target lesion using the composition for labeling the target lesion described above. More specifically, the present invention provides a method for providing information on the location of a target lesion, including the steps of directly administering a composition for labeling the target lesion in the form of microparticles containing a biocompatible polymer and a dye for biological tissue to a target lesion or the vicinity of the target lesion; and the steps of recognizing a location where the color of the tissue to which the composition for labeling the target lesion has been administered changes as the target lesion and confirming it in real time during surgery on the labeled area.

When the composition for labeling a target lesion provided in the present invention is administered to a lesion tissue in a biological body, the location of the lesion can be labeled using color, near-infrared fluorescence, or a combination thereof, and by detecting the label, the location, size, etc. of the target lesion can be detected in real time during surgery, thereby improving the accuracy in surgically removing the target lesion and preventing excessive loss of normal tissue.

In addition, since the composition for labeling a target lesion of the present invention contains a biocompatible polymer, it can remain for a long period of time while showing a high fluorescent signal in the injected lesion in the body, compared to a complex in which a dye for biological tissue is combined with another material, so that the accuracy of surgical resection of the target lesion can be easily confirmed. For example, the composition of the present invention is injected into the location of a micro-lesion before surgery, and the lesion site can be stably and accurately confirmed with the naked eye or through a fluorescent camera, etc. even several days to several weeks after surgery.

**Mode for Implementation of the Invention**

Hereinafter, the claims of this specification will be described in more detail with reference to the attached drawings and embodiments. However, the drawings and embodiments presented in this specification may be modified in various ways by those skilled in the art and may have various forms, and therefore, the descriptions in the present invention should not be considered to limit the present invention to a specific disclosed form, but to include all equivalents or substitutes included in the spirit and technical scope of the present invention. In addition, the attached drawings are presented to help those skilled in the art understand the present invention more accurately, and may be depicted in an exaggerated or reduced form compared to reality.

**{Embodiments and Evaluation}**

**<Embodiments>**

**Embodiment 1**

1) Preparation of a solution containing human serum albumin (HSA) combined with indocyanine green (ICG)

Prepare a 3.2 mM solution by dissolving 25 mg of indocyanine green (ICG) in 10 m$\ell$ of distilled water. Transfer 312.5 $\mu\ell$ of this 10 m$\ell$ solution to a 1.5 m$\ell$ Eppendorf tube and dilute with 687.5 $\mu\ell$ of distilled water to prepare 1 m$\ell$ of a 0.1 mM solution containing 78 $\mu$g of ICG. Meanwhile, prepare 0.4 m$\ell$ of a 20 % human serum albumin (HSA) solution and dilute with 0.6 m$\ell$ of distilled water to prepare an 8 % solution with a volume of 1 m$\ell$. When 125 $\mu\ell$ of each of the two solutions, 1 m$\ell$ of 0.1 mM ICG and 1 m$\ell$ of 8% HSA, are transferred to a single 1.5 m$\ell$ Eppendorf tube, a final 0.25 m$\ell$ solution containing 50 $\mu$m ICG and 4 % HSA is prepared.

2) Preparation of polymethyl methacrylate (PMMA) solution

ACRYPET polymethyl methacrylate beads (PMMA beads, Lotte MRC) (300 mg (15 pieces)) were completely dissolved in 3 m$\ell$ of dichloromethane (DCM) solution.

3) Preparation of polyvinylpyrrolidone (PVP) suspension stabilizer solution

4 g of polyvinylpyrrolidone (PVP) was completely dissolved in 100 m$\ell$ of distilled water to prepare a suspension stabilizer solution with a concentration of 4 %.

4) Preparation of polymethyl methacrylate (PMMA) microsphere particles conjugated with indocyanine green (ICG)-human serum albumin (HSA)

Place 0.25 m$\ell$ of the ICG-HSA aqueous solution prepared in the above preparation example 1) and 3 m$\ell$ of the PMMA solution prepared in the above preparation example 2) in a 15 m$\ell$ tube and mix the aqueous solution layer and the organic solution layer vigorously at room temperature and 7,500 rpm for 20 minutes using a homogenizer. Add this mixture to 40 m$\ell$ of the suspension stabilizer solution stirred at room temperature and 900 rpm. After stirring for 90 minutes to allow the organic solvent gas to escape, divide the mixture into 2 m$\ell$ Eppendorf tubes and wash three times at 2,000 rpm for 10 minutes using a centrifuge. All dispersed ICG-HSA-PMMA microspheres are combined in one tube. This material is filtered through 70 $\mu$m and 30 $\mu$m size filters to obtain a result with a size of 30 to 60 $\mu$m. After filtering, the powdered ICG-HSA-PMMA dried at room temperature is placed in a gamma irradiator and sterilized at 75 Gy for 17 seconds.

**Embodiment 2**

In the process for preparing a polyvinylpyrrolidone (PVP) suspension stabilizer solution of Embodiment 1 above, 4 g of polyvinylpyrrolidone (PVP) was completely dissolved in 100 m$\ell$ of distilled water to prepare a 4% concentration polyvinylpyrrolidone (PVP) solution, and then 0.15 g of hyaluronic acid was added to prepare a suspension stabilizer solution, and the composition for labeling the target lesion was prepared in the same manner as in Embodiment 1, except that 0.15 g of hyaluronic acid was added to prepare a composition for labeling a target lesion (hereinafter referred to as "Embodiment 2").

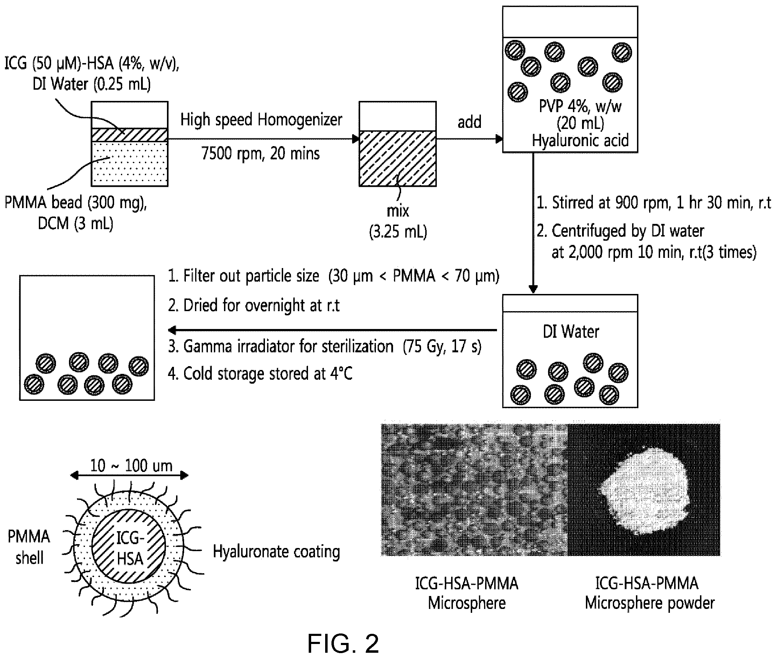

The process of the above example is illustrated in FIG. 2. FIG. 2 is a schematic diagram specifically showing the method for preparing a biocompatible composition for labeling a target lesion of the present invention. With reference to the above-described embodiment and FIG. 2, a biocompatible composition for labeling a target lesion of the present invention was prepared.

{Evaluation}

In the following, each evaluation was performed using composition for labeling the target lesion prepared according to Embodiment 2 above.

### 1. Property Evaluation of ICG-HSA-PMMA

### 1-1. Property Evaluation

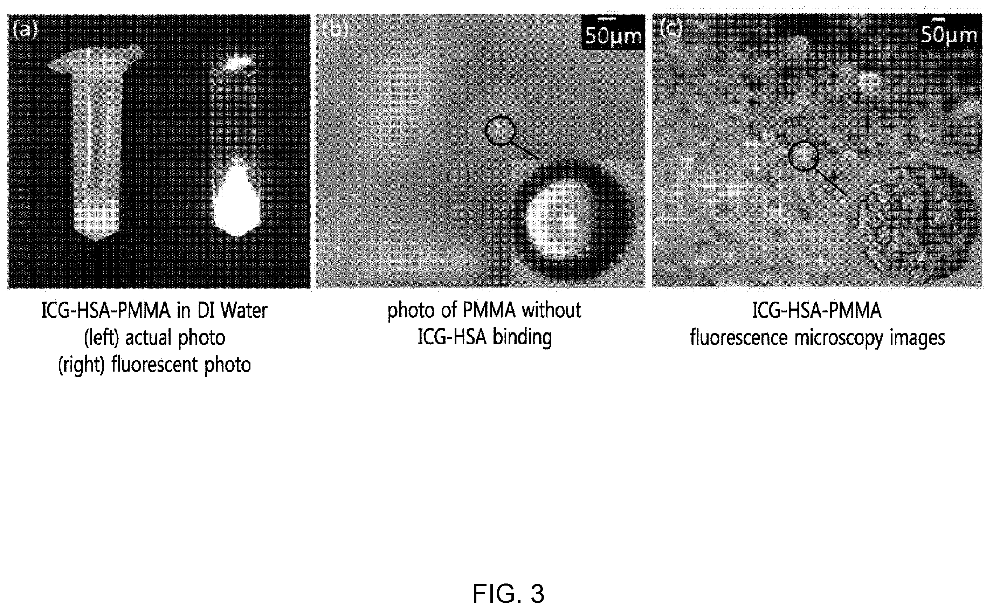

FIG. 3 shows actual photographs of PMMA including ICG and HAS, photographs taken with a fluorescence camera, and photographs taken with a fluorescence microscope of PMMA with or without ICG-HAS binding. More specifically, (a) of FIG. 3 shows an actual photograph of PMMA including ICG and HSA and photographs taken with a fluorescence camera, (b) of FIG. 3 shows a fluorescence microscopy image of PMMA without ICG-HSA binding. (c) of FIG. 3 shows a fluorescence microscopy image of ICG-HSA-PMMA.

Referring to (a) to (c) of FIG. 3, it can be confirmed that the composition for labeling a target lesion of the present invention exhibits fluorescence, and further, since the composition includes a biocompatible polymer (PMMA), it can be confirmed that it exhibits spherical microparticles.

### 1-2. Density Evaluation

Since it is difficult to directly measure the density in an aqueous solution state in an actual use form, the density of the microspheres was measured indirectly by referring to the change in density according to concentration. The density was measured using the representative ICG 6.5 $\mu$M 4 % HSA microspheres manufactured above, and the density was indirectly measured using the microspheres with a concentration of about 20 mg/m$\ell$ to 200 mg/m$\ell$ (2 to 20 %(w/v)) as follows.

More specifically, at room temperature, after filling 20 m$\ell$ of distilled water in a transparent container, the above-described microspheres were injected, and the changing density of the aqueous solution containing the microspheres was measured, and in particular, the density changing according to the concentration of the microspheres was measured. For reference, after injecting the microspheres into distilled water, it was confirmed that they did not easily dissolve in distilled water and that when shaken and mixed, they had a white suspension, and if the measurement was not performed quickly, the particles began to sink to the bottom, and after several minutes, all the particles settled.

Table 1 shows the density of distilled water containing microparticles, and describes the density according to the concentration of the microparticles contained therein.

[Table 1]

| Microsphere % (w/v) | Density (g/m$\ell$) |
|---|---|
| 25.23 | 1.0621 |
| 18.66 | 1.0469 |
| 12.61 | 1.0309 |
| 10.69 | 1.0251 |
| 9.525 | 1.0231 |
| 5.35 | 1.0112 |
| 2.16 | 1.0021 |

Referring to Table 1, a linear regression equation was obtained through linear regression analysis, and the density at 100 % was measured through extrapolation.

[Formula 1]

$$\text{density} = 0.0026 \times \text{microsphere concentration } (\%\ \text{g/m}\ell) + 0.09974$$

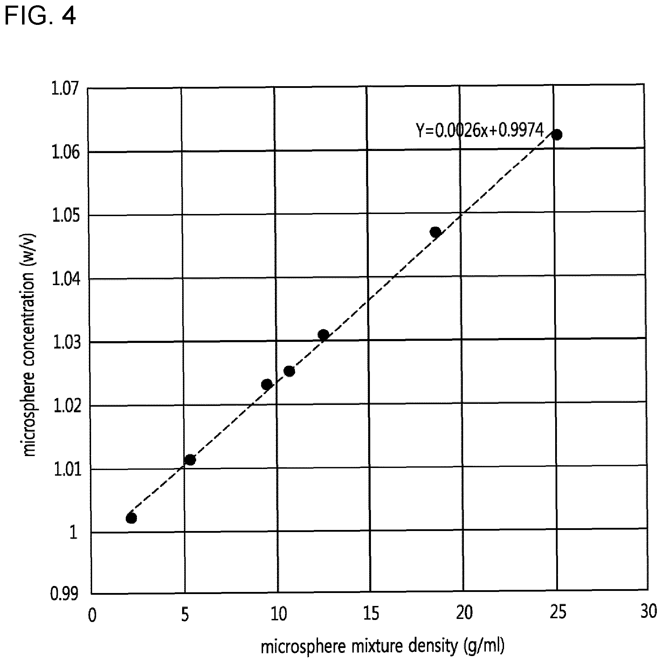

FIG. 4 is a graph showing the density according to the concentration of the microsphere particles of the present invention for distilled water. Referring to Figure 4, the density of the microspheres was estimated to be 1.2574 g/m$\ell$ according to the above-described formula. In addition, when checking the distilled water in which the microspheres were dissolved after 20 minutes, it was confirmed that a white suspension had settled at the bottom of the container. As a result, the density of the microspheres was determined to be 1.2574 g/m$\ell$, which is higher than the density of distilled water at room temperature (0.99821 g/mℓ).

**1-3. Fluorescence evaluation**

To determine the optimal ICG and albumin concentrations of the ICG-Albumin-PMMA microspheres that exhibit near-infrared fluorescence, the near-infrared fluorescence of microspheres prepared with various concentrations of ICG and albumin was measured.

In order to determine the mixing ratio of ICG and albumin that can exhibit the strongest near-infrared fluorescence, ICG at concentrations of 1.3 to 1032 μM) and albumin at concentrations of 0 to 1 % w/v (10 mg/mℓ) were reacted at various ratios to produce ICG-albumin-PMMA microspheres, and the signal intensity of near-infrared fluorescence appearing in the produced microspheres was measured, and the results are shown in Table 2.

[Table 2]

| ICG μM | Albumin (% w/v) | | | | | | | |
|---|---|---|---|---|---|---|---|---|
| | 0 | 0.05 | 0.25 | 0.5 | 0.7 | 1 | 10 | 20 |
| 1.3 | 6 | 14 | 79 | 99 | 140 | 177 | 467 | 577 |
| 3.9 | 40 | 18 | 123 | 153 | 197 | 293 | 730 | 953 |
| 6.5 | 71 | 13 | 148 | 170 | 240 | 327 | 767 | 974 |
| 9 | 104 | 11 | 152 | 154 | 224 | 284 | 574 | 734 |
| 12.9 | 137 | 9 | 121 | 125 | 143 | 193 | 330 | 484 |
| 25.8 | 153 | 4 | 85 | 90 | 113 | 150 | 200 | 317 |
| 38.7 | 142 | 5 | 54 | 60 | 77 | 107 | 133 | 157 |
| 51.6 | 133 | 3 | 34 | 42 | 50 | 83 | 97 | 117 |
| 64.5 | 125 | 5 | 25 | 32 | 40 | 60 | 64 | 84 |
| 77.4 | 111 | 5 | 18 | 25 | 33 | 37 | 37 | 50 |
| 103 | 80 | 8 | 13 | 20 | 27 | 31 | 33 | 37 |
| 258 | 40 | 10 | 5 | 12 | 20 | 20 | 22 | 22 |
| 516 | 24 | 4 | 1 | 8 | 15 | 16 | 18 | 20 |
| 774 | 13 | 2 | 1 | 7 | 11 | 14 | 17 | 16 |
| 1032 | 10 | 2 | 1 | 6 | 7 | 8 | 9 | 8 |

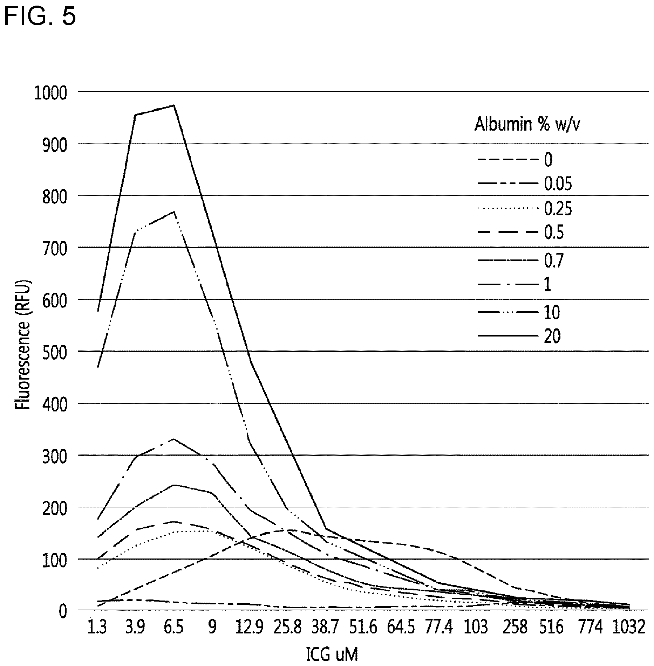

The results of Table 2 above are shown in a graph in FIG. 5. FIG. 5 is a graph showing the change in the near-infrared fluorescence signal intensity of microspheres according to the change in the concentration of ICG and albumin.

Referring to Table 2 and FIG. 5 above, when albumin was not added, the intensity of near-infrared fluorescence of the microspheres was confirmed to be the highest value of near-infrared fluorescence signal intensity at 25.8 μM) of ICG, and when 0.05 % w/v (0.5 mg/mℓ) of albumin was treated, 3.9 μM) of ICG showed the highest value of near-infrared fluorescence signal intensity, but it could be confirmed that the overall fluorescence was significantly low. When 0.25 % w/v (2.5 mg/mℓ) of albumin was treated, 9 μM) of ICG showed the highest near-infrared fluorescence signal intensity, and when the concentration of albumin was higher, which means when 0.5 % w/v (5 mg/mℓ), 0.7 % w/v (7 mg/mℓ), 1 % w/v (10 mg/mℓ), 10 % w/v (100 mg/mℓ), and 20 % w/v (200 mg/mℓ) of albumin were treated, 6.5 μM) of ICG showed the highest near-infrared fluorescence signal intensity.

Based on the above experimental results, the concentration of ICG with the strongest fluorescence intensity at the same albumin concentration was 6.5 to 25.8 μM. Therefore, in the present invention, the ICG concentration range is preferably 1 to 100 μM, and more preferably 3 to 30 μM.

In addition, at the same ICG concentration, the intensity of fluorescence continued to increase up to the highest albumin concentration used in the experiment, 20 % w/v. In reality, since the maximum concentration of serum albumin required for microsphere production is 20 % w/v, microspheres could not be produced at higher albumin concentrations. Therefore, the concentration of albumin that maximizes the intensity of fluorescence could not be determined based on the experimental results, but the experimental results confirmed that the maximum fluorescence began to increase from microspheres containing 0.5 % of albumin compared to microspheres not containing albumin. Therefore, albumin used for microsphere production is ideal at 0.5 % w/v or higher, and can be suitable for microsphere production up to 20 % w/v, which is the highest albumin concentration experimentally possible.

## 2. Evaluation of the usefulness of injectable compositions of ICG-HSA-PMMA

### 2-1. Evaluation of the usefulness of hyaluronic acid

In order to determine the usefulness of hyaluronic acid as an injectable composition, the suspension retention time of the ICG-Albumin-PMMA/hyaluronic acid composition according to the concentration of hyaluronic acid was measured.

ICG-Albumin-PMMA was used at a concentration of 2 mg/mℓ, and the final injection of ICG-Albumin-PMMA microspheres showed the appearance of a very light green or white suspension when completely dissolved in the hyaluronic acid composition.

More specifically, the time for which the ICG-Albumin-PMMA injectable composition maintained suspension after it was prepared was measured, and the time for which the suspension was maintained according to the concentration of hyaluronic acid added was measured. Here, the suspension retention time was defined as the time at which a distinction between the settled region and the floating region could be observed, based on a 20 % transmittance of 500 nm light as the criterion. The results are described in Table 3.

Table 3 shows the time for which the suspension is maintained after the ICG-Albumin-PMMA microsphere injection is prepared, according to the concentration of the hyaluronic acid solvent.

[Table 3]

| Concentration of hyaluronic acid solvent (%, w/v) | Suspension retention time |
| --- | --- |
| 0 (simple drinking water, distilled water) | Within 10 minutes |
| 0.1 | Within 60 minutes |
| 0.2 | Within 3 hours |
| 0.3 | Within 4 hours |
| 0.4 | Within 12 hours |
| 0.5 | Within 24 hours |
| 1.0 | More than 24 hours |
| 1.5 | More than 24 hours |
| 2.0 | More than 24 hours |
| 2.5 | More than 24 hours |

Referring to Table 3, it was confirmed that the injectable composition without added hyaluronic acid had a relatively shorter suspension retention time than the composition with added hyaluronic acid, and that the suspension retention time became longer as the ratio of added hyaluronic acid increased.

Meanwhile, when added to a 0.1 % hyaluronic acid injection composition, the suspension retention time increased to within 60 minutes, but the suspension retention time was 1 to 3 hours when the hyaluronic acid concentration was 0.2 % (w/v), and it was confirmed that the suspension was over 3 hours when the hyaluronic acid concentration was 0.3 % (w/v). In actual clinical practice, since the time for preparing the injection and actually injecting it is less than 1 hour, it was determined that it was effective as an actual injection preparation starting from 0.2 % (w/v).

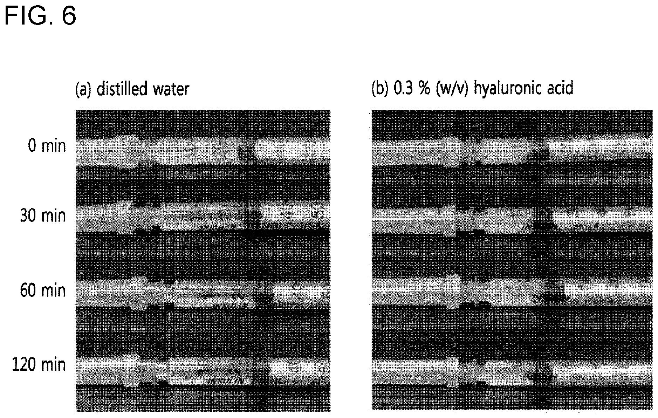

FIG. 6 is a photograph showing the degree of dispersion of microsphere particles according to the concentration of hyaluronic acid in the biocompatible injection composition for labeling target lesion of the present invention. More specifically, the photographs were taken immediately after preparation and then at 30, 60, and 120 minutes for the ICG-HSA-PMMA/hyaluronic acid injection produced as described above: (a) on the left shows the injection with microsphere mixed in water for injection (distilled water), and (b) on the right shows a microsphere injection prepared with 0.3 % hyaluronic acid.

Referring to FIG. 6, it was found that the injectable composition prepared in the injection water quickly sank, whereas the injectable composition with 0.3% hyaluronic acid added did not completely sink for a long time (up to 120 minutes). That is, from the results of FIG. 3, it can be concluded that the ICG-HSA-PMMA/hyaluronic acid injectable composition with 0.3 % hyaluronic acid added to the microsphere particles is very useful as an injectable composition because it will not quickly sink during injection preparation.

## 2-2. Evaluation of usefulness as an injectable composition

The gliding force (N) on the finger during injection was measured under different conditions depending on the concentration of hyaluronic acid, and the usefulness of ICG-Albumin-PMMA/hyaluronic acid as an injectable composition was determined.

More specifically, when the final prepared injection was used as an injection, the ease of injection was evaluated using a 3 cc disposable syringe (3 cc, BD, Luer-Lock Tip, Singapore) equipped with a 21G, 22G, 23G, and 26G needle (Kovax, KOREAVACCINE CO., LTD.), a 20G spinal needle (acupuncture needle, Taechang Industrial Co., Ltd.), and a 7Fr endoscopic syringe (20G (needle inner diameter: 0.603 mm), 21G (needle inner diameter: 0.495 mm), and 22G (needle inner diameter: 0.394 mm), 23G (needle inner diameter: 0.318 mm), and 26G (needle inner diameter: 0.241 mm)).

The force applied to the syringe handle (force applied to the finger during injection, N) was measured for each needle. In addition, resistance was measured using an endoscopic syringe with a 1.8 m connecting tube (7 Fr, length 180 cm, MTW, Germany).

The injection used in the experiment used a concentration of 2 mg/m$\ell$ of ICG-Albumin-PMMA microspheres, and the concentration of hyaluronic acid in the final injection was set to 0.1 to 3.0 % (w/v). After the injection was injected into the syringe, the experiment was performed immediately to prevent the microspheres from settling, and the strain-gauge dynamometer was used to measure the strain while the final injection was injected at a speed of 0.1 cc/sec (Digital Force Ga $\mu$g e DPU-500N, IMADA, Toyohashi, Japan). The results are described in Table 4 below.

[Table 4]

| N | Blank syringe | Distilled water | Hyaluronic acid (% (w/v)) [ICG-Albumin-PMMA microspheres 2 mg/mℓ] | | | | | | | | | | | | | | |
|---|---|---|---|---|---|---|---|---|---|---|---|---|---|---|---|---|
| | | | 0.1 | 0.2 | 0.3 | 0.4 | 0.5 | 0.6 | 0.7 | 0.8 | 0.9 | 1.0 | 1.5 | 2.0 | 2.5 | 3 |
| 26 G | 1.2 | 2.9 | 3.0 | 3.3 | 3.5 | 4.3 | 4.9 | 5.2 | 5.9 | 6.1 | 6.5 | 6.6 | 7.9 | 9.9 | 12.6 | 15.8 |
| 23 G | 1.9 | 2.3 | 2.4 | 2.5 | 2.7 | 3.0 | 3.5 | 3.8 | 4.1 | 4.2 | 4.4 | 4.6 | 6.5 | 8.6 | 10.2 | 12.8 |
| 22 G | 1.6 | 1.8 | 1.8 | 2.2 | 2.6 | 2.7 | 3.1 | 3.3 | 3.6 | 3.9 | 3.8 | 4.0 | 6.6 | 8.3 | 9.1 | 11.8 |
| 21 G | 1.8 | 1.9 | 1.9 | 2.2 | 2.5 | 2.7 | 2.7 | 2.9 | 3.1 | 3.2 | 3.4 | 3.5 | 4.6 | 6.6 | 7.9 | 9.5 |
| 20 G | 1.8 | 1.7 | 1.8 | 2.5 | 3.1 | 3.4 | 3.5 | 3.8 | 3.9 | 4.5 | 4.7 | 4.9 | 8.4 | 12.8 | 17.6 | 19.6 |
| 7Fr | 1.9 | 3.3 | 3.4 | 7.5 | 12. 1 | 17. 8 | 21. 6 | 25. 4 | 27. 2 | 33. 4 | 36. 9 | 39. 5 | Un measu rable | Un measu rable | Un measu rable | Un measu rable |

In general, when giving an injection in clinical practice, a force of 0.6 to 1.4 N is applied to the finger during the injection. The grip strength of an average adult is 400 N, and the grip strength that can be handled by the finger is weaker than this, and much less force is required to make delicate adjustments such as controlling the injection amount. A force gauge was installed between the thumb and index/middle finger in 10 men and women, and the maximum power (MaxPower) when applying the maximum force and the power when injecting comfortably (EasyPower) were measured. The maximum power (MaxPower) was 98.1 N (113.5 to 78.2, standard deviation 9.8) for adult males and 43.1 N (50.6 to 34.2, standard deviation 6.1) for adult females, and 25.4 N (29.7 to 20.1, standard deviation 3.3) for male adults and 14.5 N (18.3 to 11.5, standard deviation 2.2) for female adults when the force was applied comfortably. The ideal resistance of the injection was determined based on the maximum pressure of 43 N and the comfort level of 14.5 N for female adults. In other words, it was determined that the maximum force that can be injected was 40 N and the ideal resistance for comfortable injection was 10 N.

When using a general injection needle (21 to 26G), the resistance was not larger than 10 N for injection up to 1% hyaluronic acid concentration, so there was no difficulty in use. Even for a puncture needle with a diameter of 20G and a much longer needle than other needles, the resistance did not exceed 10 N at 1 %.

On the other hand, when using an endoscopic syringe needle (7 Fr, 180 cm), which requires a lot of resistance due to the long tube length, the injection water required a force of 1.9 N, and when the concentration of hyaluronic acid was 1 % (w/v), the force was 39.5 N, so a significantly high pressure was required to inject, making it impossible to control the injection amount or speed.

Although there are individual differences, when a force of 40 N is applied to the finger, it is difficult to inject into the tissue, making it difficult to inject precisely. On the other hand, when an endoscopic syringe needle (7 Fr, 180 cm) was used, the resistance increased (> 40 N) when hyaluronic acid exceeded 1%, making it impossible to inject with bare hands.

Under conditions where hand injection is possible with maximum force (resistance less than 40 N), it was possible to inject with hyaluronic acid concentration up to 3 % (w/v), and even with a long needle (20 G), up to 3 % (w/v) was possible. Even with an extremely long special syringe connected by a conduit from the syringe to the needle (endoscopic syringe), it was possible to inject without difficulty of up to 1.0 % (w/v).

Meanwhile, when using general syringes (26G to 20G) that come into direct contact with tissues, it is preferable to inject smoothly into the tissue, and it is ideal to apply a force of less than 10 N. For hyaluronic acid, injections with general needles (21G to 26G) could be performed with a force of less than 10 N for up to 2 % (w/v), and with puncture needles (20G) for concentrations up to 1.5 % (w/v). Endoscopic syringes could be used without difficulty for concentrations up to 0.2 to 0.3 % (w/v).

### 2-3. Evaluation of syringe pressure changes according to sedimentation

The pressure (A) at the time of immediate injection of the injectable composition to which hyaluronic acid is added, the pressure (B) at the beginning of the injection after the composition has settled down after 120 minutes of standing, and the pressure (C) at the middle of the injection are measured and are shown in Table 5 below.

The injectable used in the experiment was ICG-Albumin-PMMA microspheres at a concentration of 2 mg/mℓ, and the concentration of hyaluronic acid in the final injection was 0.1 to 3.0 % (w/v). The syringe was 26G, and the unit of pressure applied to the syringe was measured in N. In addition, the gliding force was measured in units of N (Digital Force Ga μg e DPU-500N, IMADA, Toyohashi, Japan).

[Table 5]

| 26G | Distilled water | hyaluronic acid (% (w/v)) | | | | | | | | | | | | | |
|---|---|---|---|---|---|---|---|---|---|---|---|---|---|---|---|
| | | 0.1 | 0.2 | 0.3 | 0.4 | 0.5 | 0.6 | 0.7 | 0.8 | 0.9 | 1.0 | 1.5 | 2.0 | 2.5 | 3 |
| A | 2.9 | 2.9 | 3.1 | 3.5 | 4.2 | 4.7 | 5.3 | 5.8 | 6.2 | 6.3 | 6.7 | 7.7 | 9.7 | 12.4 | 15.6 |
| B | ND | 45.2 | 34.7 | 3.7 | 4.3 | 4.8 | 5.4 | 5.7 | 6.3 | 6.5 | 6.9 | 7.5 | 9.8 | 12.2 | 15.8 |
| C | ND | 3.5 | 3.6 | 3.5 | 4.2 | 4.3 | 5.2 | 5.3 | 6.0 | 6.2 | 6.5 | 7.3 | 9.5 | 12.5 | 15.8 |
| B-A | ND | 42.3 | 31.6 | 0.2 | 0.1 | 0.1 | 0.1 | -0.1 | 0.1 | 0.2 | 0.2 | -0.2 | 0.1 | -0.2 | 0.2 |

Referring to Table 5, it can be seen that the pressure (A) when the injectable composition was injected immediately and the pressure (B) applied to the syringe after leaving the injectable composition for 120 minutes and precipitating the composition, the higher the concentration of hyaluronic acid, the higher the pressure (A, B). The difference between A and B was almost insignificant after 0.3 % (-0.2 to 0.2 N). As the concentration of hyaluronic acid increased above 0.3 %, there was little difference between the pressure (B) applied to the syringe after leaving the injectable composition for 120 minutes and the pressure (A) when the injectable composition was injected immediately. The average B-A value was 0.07 N from the hyaluronic acid concentration of 0.3 to 3.0 % (w/v), and the standard deviation was 0.15 N.

In Table 5, the data for hyaluronic acid concentrations of 0.1 % and 0.2 % show that the pressure after 2 hours of instillation was 45.2 and 34.7 N, which is difficult to inject, meaning that the needle is blocked by the injectant. After the injection was moved, the pressure decreased rapidly, and there was no difference from the pressure (A) when the injectable was injected directly.

In other words, by adding an appropriate amount of hyaluronic acid (more than 0.3 %), the precipitation rate could be slow down, and the disadvantage of requiring excessive force to be applied to start the injection due to the injection needle being blocked by the precipitated composition could be resolved.

Meanwhile, when the concentration of hyaluronic acid exceeds 1 % (w/v), it can be confirmed that the pressure increases upon injection due to the viscosity of hyaluronic acid. As the pressure increases, the resistance upon injection also increases, which may cause inconvenience in use as an injectable.

### 3. Stability evaluation of ICG-HSA-PMMA

PMMA microspheres conjugated with ICG and HSA, which can generate near-infrared fluorescence signals, were expected to serve as markers that work more stably in vivo. Therefore, the microsphere particles were produced and their stability was confirmed under both in vitro and in vivo conditions.

### 3-1. Confirmation of in vitro near-infrared fluorescence signal stability

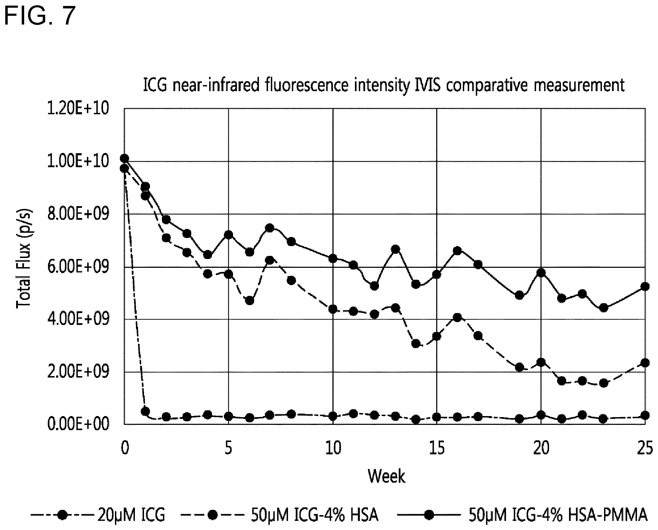

FIG. 7 is a graph showing a comparison of the change in intensity of near-infrared fluorescence signals over time for ICG, ICG-HSA, and ICG-HSA-PMMA particles under in vitro conditions, measured using IVIS (Perkin Elmer, Hopkinton, MA).

Referring to FIG. 7, according to embodiments of the present invention, it was confirmed that in the preparation of PMMA microspheres (ICGHSA-PMMA) conjugated with ICG-HSA, a suitable mixing ratio for preparing microsphere particles exhibiting a high level of near-infrared fluorescence signal was to mix 50 $\mu$M) of ICG with 4 % of HSA and mix it with 300 mg of PMMA beads. When conjugated to a biopolymer such as albumin, it shows stronger fluorescence and longer duration than when ICG alone is embedded in microspheres.

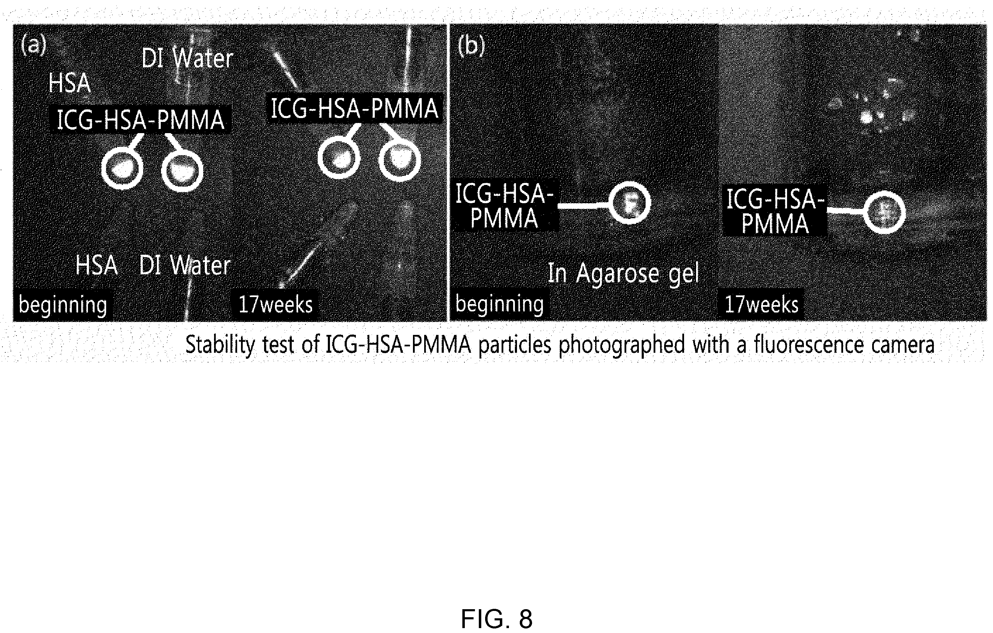

The results of measuring the intensity of the near-infrared fluorescence signal emitted by the ICG-HSA-PMMA particles prepared in the above preparation example 4 for 5 months under in vitro conditions using IVIS are shown in FIG. 8. FIG. 8 is a photograph comparing the stability of the particles over time after placing the ICG-HSA-PMMA particles in HSA, distilled water, and agarose gel, respectively. More specifically, (a) of FIG. 8 is a photograph comparing the stability of the particles over time after placing the ICG-HSA-PMMA particles in HSA and distilled water, respectively. (b) of FIG. 8 is a photograph comparing the stability of the particles over time after placing the ICG-HSA-PMMA particles in agarose gel.

Referring to (a) of FIG. 8, the particles containing PMMA showed stability with a relatively small decrease in the intensity of the near-infrared fluorescence signal. In addition, referring to (b) of FIG. 8, the prepared particles were placed in (a) 20 % HSA, distilled water, and (b) agarose gel, respectively, and the near-infrared fluorescence signal emitted for 17 weeks was measured with a fluorescence camera, confirming stability even with the naked eye.

### 3-2. Confirmation of stability of near-infrared fluorescence signal in vivo

The ICG-HSA-PMMA microspheres prepared in the above preparation example 4 were put into a hyaluronic acid solution or distilled water, and 100 $\mu\ell$ was injected into the backs of ICR mice, respectively, and the intensity of the near-infrared fluorescence signal generated in each mouse for 4 months was measured using IVIS and fluorescence camera equipment.

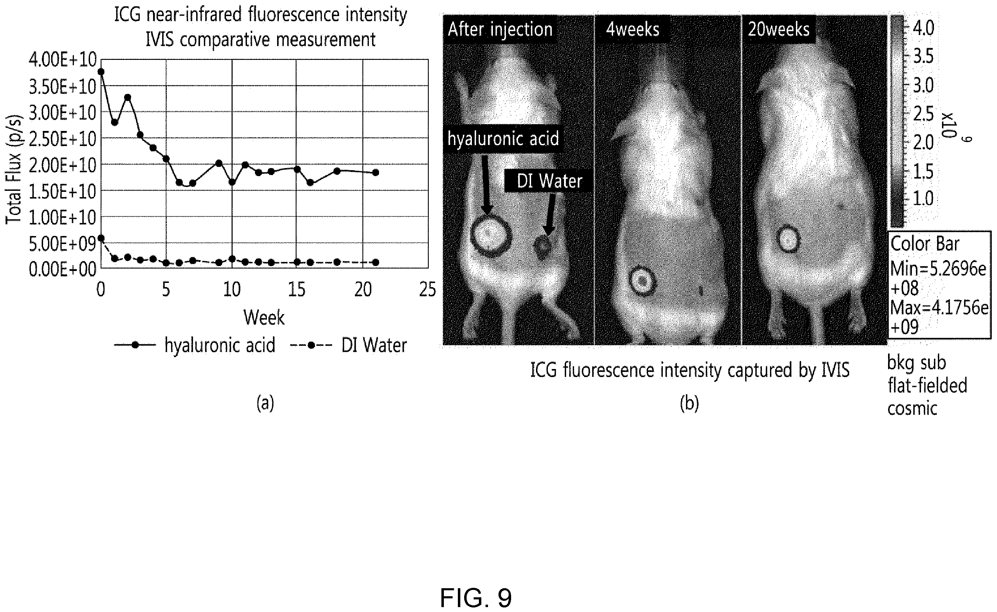

FIG. 9 is a comparison of the change in intensity of the near-infrared fluorescence signal over time when ICG-HSA-PMMA particles injected into ICR mice under in vivo conditions were injected together with hyaluronic acid or distilled water, as measured by IVIS. More specifically, (a) of FIG. 9 is a graph quantitatively showing the change in intensity of the near-infrared fluorescence signal over time when ICG-HSA-PMMA particles injected into ICR mice were measured by IVIS, and (b) of FIG. 9 is an image taken by IVIS over time when ICG-HSA-PMMA particles injected into ICR mice.

Referring to FIG. 9, IVIS observations showed that the ICG-HSA-PMMA/hyaluronic acid mixture remained stable with a high intensity near-infrared fluorescence signal, while the mixture in distilled water showed a very low fluorescence signal.

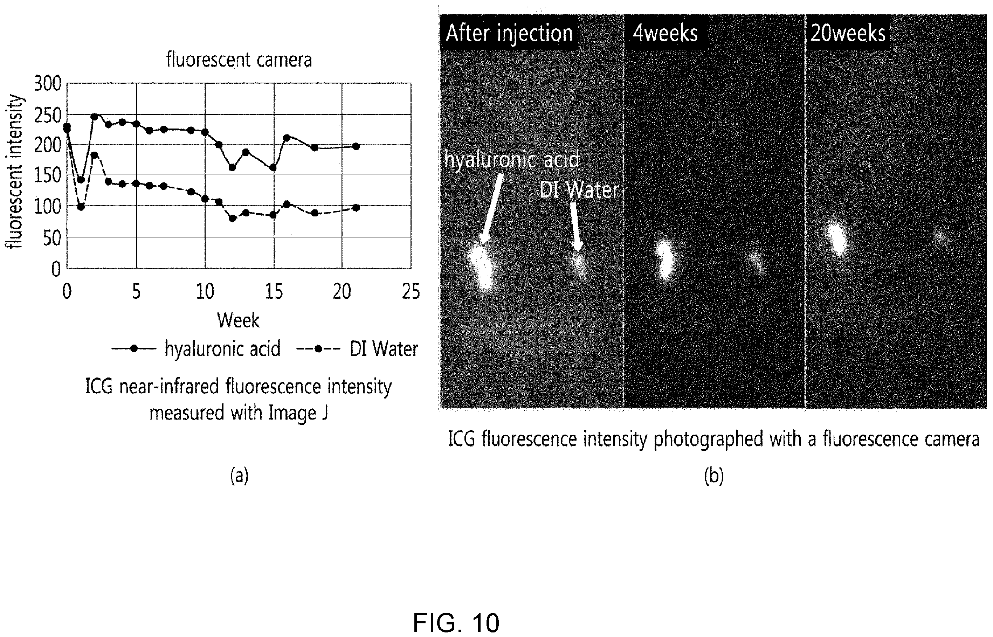

FIG. 10 is a graph showing the change in intensity of the near-infrared fluorescence signal over time when ICG-HSA-PMMA particles were injected into ICR mice in vivo, together with hyaluronic acid or distilled water, and analyzed using the Image J program. It is also a result of taking pictures of the mouse with the fluorescence camera. More specifically, (a) of FIG. 10 is a graph showing quantitative changes in the intensity of the near-infrared fluorescence of ICG-HSA-PMMA particles injected into ICR mice over time, and (b) of FIG. 10 is an image taken with a fluorescence camera over time of ICG-HSA-PMMA particles injected into ICR mice.

Referring to FIG. 10, in the case of the fluorescent camera, the fluorescence intensity was measured using the Image J program after shooting, and both mixtures showed high-intensity near-infrared fluorescence signals, but over time, the ICG-HSA-PMMA/hyaluronic acid mixture maintained stability, while the distilled water mixture showed a gradually decreasing fluorescence signal.

In addition, referring to FIGS. 9 and 10, when ICG-HSA-PMMA microspheres are mixed with a hyaluronic acid solution, it can be confirmed that the in vivo tissue retention of the microsphere particles is improved by the solution. The hyaluronic acid plays a role in gathering the complexes injected into the body together and increasing the fluorescence signal, thereby preventing the complexes injected into the body from spreading as much as possible and obtaining a high fluorescence signal for several months. Therefore, the injection composition of the complex of the present invention may additionally include hyaluronic acid.

The composition for labeling the target lesion of the present invention, when injected into the target lesion, remains continuously for several months and can detect the location and size of the labeled lesion in real time during surgery, so it not only improves the success rate of surgical operation of the target lesion but also prevents excessive loss of normal tissue, so it can be widely used as an effective surgical marker. In the experiment, only the results of the images taken at a maximum of 20 weeks were disclosed, but this was due to other circumstances such as the termination of the experiment according to the animal welfare-related experimental regulations, and it was confirmed that the fluorescence of the ICG-HAS-PMMA microspheres was well maintained up to 25 weeks. In addition, although it is not shown in the graph, it was confirmed that the fluorescence of the microspheres lasted for more than a year in vitro.

The above description is merely an example of the technical idea of the present invention, and those skilled in the art will appreciate that various modifications and variations may be made without departing from the essential characteristics of the present invention.

Therefore, the embodiments disclosed in the present invention are not intended to limit the technical idea of the present invention but to explain it, and the scope of the technical idea of the present invention is not limited by these embodiments. The protection scope of the present invention should be interpreted by the following claims, and all technical ideas within the equivalent scope should be interpreted as being included in the scope of the rights of the present invention.

**Industrial Applicability**

The composition for labeling a target lesion according to the present invention, when injected into a target lesion, remains there for several months and can detect the location and size of the labeled lesion in real time during surgery, thereby not only improving the success rate of surgical operation for the target lesion, but also preventing excessive loss of normal tissue, and therefore can be widely utilized as an effective surgical marker.

**Claims**

1. A composition for labeling a target lesion,

   in form of microspheres comprising:

   a biocompatible polymer; and
   a dye for biological tissue,

   and directly administered to a target lesion to confirm size and location of the target lesion in real time during surgery for a target site.

2. The composition of claim 1,
   wherein the microsphere further comprises a surface modifier coated on surface of the microsphere.

3. The composition of claim 1,
   wherein the biocompatible polymer is one of polymethylmethacrylate (PMMA), polyglycolic acid (PGA), polylactic acid (PLA), polylactic acid-polyglycolic acid copolymer (PLGA), poly-ε-caprolactone (PCL), lactic acid-ε-caprolactone copolymer (PLCL), polydioxanone (PDO), polytrimethylene carbonate (PTMC), polyamino acid, polyanhydride, polyorthoester, polyphosphazene, polyiminocarbonate, polyphosphoester, polyhydroxyvalate, copolymers thereof, and mixtures thereof.

4. The composition of claim 1,

wherein the dye for biological tissue is a visible dye or a fluorescent dye.

5.  The composition of claim 4,
    wherein the visible dye is one selected from a group of neutral red, Nile blue, Bismarck brown, lithium carmine, trypan blue, Janus green, methyl violet, auramine, malachite green, safranin, eosin, Congo red, erythrosine, nigrosine, Alcian blue hematoxylin, aniline blue, light green, and combinations thereof.

6.  The composition of claim 4,
    wherein the fluorescent dye is a near-infrared fluorescent dye.

7.  The composition of claim 4,
    wherein the fluorescent dye is an indocyanine green (ICG).

8.  The composition of claim 4,
    wherein the fluorescent dye is used alone or in conjugation with biological protein.

9.  The composition of claim 2,
    wherein the surface modifier is one of hyaluronic acid, collagen, and a mixture thereof.

10. The composition of claim 1,
    wherein the microsphere further comprises biological protein.

11. The composition of claim 10,
    wherein the biological protein is albumin.

12. The composition of claim 1,
    wherein the microsphere has a diameter of 10 to 100 $\mu$m.

13. The composition of claim 1,
    wherein the composition has an average density of 1 to 3 g/m$\ell$.

14. A method of preparing a composition for labeling a target lesion, comprising:

    mixing an organic solvent containing a biocompatible polymer and distilled water containing a dye for biological tissue; and
    adding the mixed solution to a water-soluble solvent and stirring to form microspheres.

15. The method of claim 14, further comprising:
    adding a surface modifier to the water-soluble solvent.

16. The method of claim 14, further comprising:
    washing and filtering the stirred solution to select microspheres of a specific size.

17. The method of claim 14,
    wherein the distilled water further contains a complex of dye for biological tissue-biological protein.

18. The method of claim 14,
    wherein the organic solvent is one selected from a group of dichloromethane (DCM), o-xylene, m-xylene, p-xylene, and combinations thereof.

19. The method of claim 14,
    wherein a concentration of the dye for biological tissue is 1 to 100 $\mu$M.

20. The method of claim 14,
    wherein the water-soluble solvent is one selected from a group of polyvinylpyrrolidonem (PVP), polyacrylic acid (PAA), polyacryl amide (PAAm), polyhydroxyethyl methacrylate (PHEMA), polyvinyl alcohol, (PVA), and combinations thereof.

21. An injectable composition for labeling a target lesion, comprising:

   a composition for labeling a target lesion in form of microspheres comprising a biocompatible polymer and a dye for biological tissue; and
   hyaluronic acid (HA) having an average molecular weight of 0.5 to 3.0 MDa.

22. The injectable composition of claim 21,
   wherein with respect to 100 wt% of the injectable composition, hyaluronic acid is included in an amount of 0.3 to 1 wt%.

23. The injectable composition of claim 21,
   wherein a target lesion injected with the injectable composition remains labeled for 4 to 12 months.

24. A method of providing information on location of a target lesion, comprising:

   directly administering a composition for labeling a target lesion in form of microspheres comprising a biocompatible polymer and a dye for biological tissue to a target lesion or around the target lesion; and
   recognizing a location of color change in a tissue to which the composition for labeling a target lesion as the target lesion to confirm a size and a location of the target lesion in real time during surgery.

ICG-HSA,
DI Water

High speed Homogenizer
7500 rpm, 20 mins

mix

add

Hyaluronic acid,
PVP

PMMA, DCM

1. Stirred at room temperature
2. Centrifuged adding DI water

1. Filter out particle size
2. Dried for overnight at r.t
3. Gamma irradiator for sterilization
4. Cold storage stored at 4°C

DI Water

10 ~ 100 um

PMMA
shell

ICG-
HSA

Hyaluronate coating

ICG-HSA-PMMA
Microsphere

ICG-HSA-PMMA
Microsphere powder

FIG. 1

FIG. 2

FIG. 3

FIG. 4

FIG. 5

FIG. 6

(a) distilled water                    (b) 0.3 % (w/v) hyaluronic acid

FIG. 7

ICG near-infrared fluorescence intensity IVIS comparative measurement

---●--- 20μM ICG    --●-- 50μM ICG-4% HSA    ——●—— 50μM ICG-4% HSA-PMMA

Stability test of ICG-HSA-PMMA particles photographed with a fluorescence camera

FIG. 8

FIG. 9

fluorescent camera

ICG near-infrared fluorescence intensity
measured with Image J

(a)

ICG fluorescence intensity photographed with a fluorescence camera

(b)

FIG. 10

# INTERNATIONAL SEARCH REPORT

| | International application No. |
|---|---|
| | **PCT/KR2023/000601** |

## A. CLASSIFICATION OF SUBJECT MATTER

**A61K 49/00**(2006.01)i; **A61B 90/00**(2016.01)i

According to International Patent Classification (IPC) or to both national classification and IPC

## B. FIELDS SEARCHED

Minimum documentation searched (classification system followed by classification symbols)

A61K 49/00(2006.01); A61B 5/00(2006.01); A61B 6/03(2006.01); A61K 31/496(2006.01); A61K 47/36(2006.01); A61K 49/04(2006.01); A61K 9/14(2006.01); A61K 9/16(2006.01); A61K 9/50(2006.01); A61L 31/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생체적합성 고분자(biocompatible polymer), 염료(dye), 미립구(microparticle), 표지자(marker), 수술 부위(surgical region), 실시간(real-time), 히알루론산(hyaluronic acid)

## C. DOCUMENTS CONSIDERED TO BE RELEVANT

| Category* | Citation of document, with indication, where appropriate, of the relevant passages | Relevant to claim No. |
|---|---|---|
| X | KR 10-2020-0052223 A (NATIONAL CANCER CENTER) 14 May 2020 (2020-05-14)<br>See claim 12; and paragraphs [0001]-[0052] and [0091]-[0232]. | 1-24 |
| A | US 10986997 B2 (MEMORIAL SLOAN KETTERING CANCER CENTER et al.) 27 April 2021 (2021-04-27)<br>See entire document. | 1-24 |
| A | US 2021-0007990 A1 (BROWN UNIVERSITY et al.) 14 January 2021 (2021-01-14)<br>See entire document. | 1-24 |
| A | US 2020-0206366 A1 (INCEPT, LLC) 02 July 2020 (2020-07-02)<br>See entire document. | 1-24 |
| A | US 2014-0271897 A1 (PATHAK HOLDING, LLC) 18 September 2014 (2014-09-18)<br>See entire document. | 1-24 |

☐ Further documents are listed in the continuation of Box C.    ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **24 April 2023** | **26 April 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/000601**

| Patent document cited in search report | | | Publication date (day/month/year) | Patent family member(s) | | | Publication date (day/month/year) |
|---|---|---|---|---|---|---|---|
| KR | 10-2020-0052223 | A | 14 May 2020 | CN | 112969479 | A | 15 June 2021 |
| | | | | EP | 3878476 | A1 | 15 September 2021 |
| | | | | JP | 2022-506417 | A | 17 January 2022 |
| | | | | KR | 10-2315855 | B1 | 22 October 2021 |
| | | | | US | 2021-0369875 | A1 | 02 December 2021 |
| | | | | WO | 2020-096281 | A1 | 14 May 2020 |
| US | 10986997 | B2 | 27 April 2021 | AU | 2014-373656 | A1 | 07 July 2016 |
| | | | | AU | 2014-373656 | A1 | 09 July 2015 |
| | | | | CA | 2935690 | A1 | 09 July 2015 |
| | | | | CN | 106455979 | A | 22 February 2017 |
| | | | | CN | 113349707 | A | 07 September 2021 |
| | | | | EP | 3089653 | A1 | 09 November 2016 |
| | | | | JP | 2017-504019 | A | 02 February 2017 |
| | | | | JP | 2019-069267 | A | 09 May 2019 |
| | | | | JP | 6681334 | B2 | 15 April 2020 |
| | | | | KR | 10-2016-0117440 | A | 10 October 2016 |
| | | | | RU | 2016127260 | A | 06 February 2018 |
| | | | | US | 2015-0182118 | A1 | 02 July 2015 |
| | | | | US | 2020-214571 | A1 | 09 July 2020 |
| | | | | WO | 2015-103420 | A1 | 09 July 2015 |
| US | 2021-0007990 | A1 | 14 January 2021 | AU | 2015-301399 | A1 | 06 April 2017 |
| | | | | AU | 2015-301399 | B2 | 08 November 2018 |
| | | | | CA | 2957966 | A1 | 18 February 2016 |
| | | | | CA | 2957966 | C | 30 November 2021 |
| | | | | EP | 3179985 | A1 | 21 June 2017 |
| | | | | JP | 2017-524743 | A | 31 August 2017 |
| | | | | JP | 6910949 | B2 | 28 July 2021 |
| | | | | US | 10722468 | B2 | 28 July 2020 |
| | | | | US | 11504329 | B2 | 22 November 2022 |
| | | | | US | 2017-273909 | A1 | 28 September 2017 |
| | | | | WO | 2016-025911 | A1 | 18 February 2016 |
| US | 2020-0206366 | A1 | 02 July 2020 | AU | 2010-340067 | A1 | 12 July 2012 |
| | | | | AU | 2010-340067 | B2 | 19 March 2015 |
| | | | | CA | 2782899 | C | 11 June 2019 |
| | | | | CA | 3042067 | C | 18 October 2022 |
| | | | | CA | 3149284 | A1 | 14 July 2011 |
| | | | | CN | 102844054 | A | 26 December 2012 |
| | | | | CN | 102844054 | B | 20 April 2016 |
| | | | | EP | 2512540 | A2 | 24 October 2012 |
| | | | | EP | 2512540 | A4 | 14 August 2013 |
| | | | | IN | 5177DEN2012 | A | 23 October 2015 |
| | | | | JP | 2013-514152 | A | 25 April 2013 |
| | | | | JP | 5820815 | B2 | 24 November 2015 |
| | | | | US | 8383161 | B2 | 26 February 2013 |
| | | | | US | 8852646 | B2 | 07 October 2014 |
| | | | | US | 9308283 | B2 | 12 April 2016 |
| | | | | WO | 2011-084465 | A2 | 14 July 2011 |
| | | | | WO | 2011-084465 | A3 | 17 November 2011 |
| US | 2014-0271897 | A1 | 18 September 2014 | US | 2015-0359896 | A1 | 17 December 2015 |
| | | | | US | 2016-0243026 | A1 | 25 August 2016 |

Form PCT/ISA/210 (patent family annex) (July 2022)

## INTERNATIONAL SEARCH REPORT
### Information on patent family members

International application No.

**PCT/KR2023/000601**

| Patent document cited in search report | Publication date (day/month/year) | Patent family member(s) | | | Publication date (day/month/year) |
|---|---|---|---|---|---|
| | | US | 9789073 | B2 | 17 October 2017 |
| | | WO | 2014-160387 | A2 | 02 October 2014 |
| | | WO | 2014-160387 | A3 | 18 December 2014 |
| | | WO | 2018-017674 | A1 | 25 January 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120153793

- KR 2011009271 W

**Non-patent literature cited in the description**

- **T. KITAI et al.** *Breast Cancer*, 2005, vol. 12, 211-215